# EUROPEAN PATENT APPLICATION

(11) **EP 4 108 674 A1**
(43) Date of publication of application: **28.12.2022**
(21) Application number: 21756344.4
(22) Date of filing: 05.02.2021
(51) Int. Cl.: C07K 1/18, B01D 15/38

(54) **OPTIMIZED METHOD FOR BEVACIZUMAB PURIFICATION**

(30) Priority: 21.02.2020 KR 20200021724
(71) Applicant: PRESTIGE BIOPHARMA PTE. LTD., Singapore 118222 (SG)
(72) Inventor: YANG, Jae Young, Cheongju-si Chungcheongbuk-do 28160 (KR); PARK, Ji Sung, Cheongju-si Chungcheongbuk-do 28222 (KR); SHIM, Jae Ho, Cheongju-si Chungcheongbuk-do 28160 (KR); UM, Yoon Joeng, Cheongju-si Chungcheongbuk-do 28160 (KR); PARK, Joo Yang, Seoul 03964 (KR)
(74) Representative: Frenkel, Matthias Alexander
(86) International application number: PCT/KR2021/001523
(87) International publication number: WO 2021/167276

(57) **Abstract**

The present invention relates to a method of purifying an antibody, which can prepare a desired antibody with high purity and high quality by removing impurities without using an expensive protein A column, and particularly, can purify an antibody in a high yield while greatly reducing an amount (volume) of a buffer used in an elution process; and an antibody prepared by the method.

## Description

### TECHNICAL FIELD

The present invention relates to a method of purifying an antibody with high purity and high yield at low cost without using a protein A column, which is affinity chromatography.

### BACKGROUND ART

The latest trend in biopharmaceutical research and development is more focused on the development of antibody fragments. Although a large number of biosimilar therapeutic proteins have been developed than ever before, significant difficulties in developing biosimilar products are the high costs of downstream processing, non-selective elimination of process and product-related impurities, proteolytic degradation of a product, and the like. Antibody fragments offer certain advantages over full size monoclonal antibody (mAb) therapeutics, such advantages include, for example, enhanced deep penetration into tumors, binding to specific epitopes which are not accessible to full size mAbs, and the like. Advances in upstream processes such as high titer clones and continuous bio-manufacturing have shifted the focus of the biopharmaceutical industry towards improving overall downstream processing economics. The downstream processing constitutes approximately 60 to 70% of the overall manufacturing cost for monoclonal antibody therapeutics. The capture, intermediate and polishing steps of downstream processing involves use of various chromatographic operations.

Accordingly, a lot of research and development on the purification process has been conducted in relation to antibody drugs.

For example, purification methods for monoclonal antibodies, typically including four basic steps are under technical development, and there are a lot of studies related to this. These steps are steps of (1) harvest - isolating host cells from a fermentation culture; (2) capture - isolating an antibody from the majority of components among purified harvested products; (3) micropurification - removing residual host cell contaminants and aggregates; and (4) formulation - disposing the antibody on a suitable carrier for maximum stability and shelf life. However, these steps do not necessarily produce an antibody composition having sufficient purity for use in pharmaceutical circumstances. Therefore, it is of utmost importance to have a method of producing and purifying a target antibody in pure form from which impurities have been sufficiently removed to be suitable for pharmaceutical use.

Specifically, as a purification step for producing an antibody drug, a purification step using a protein A column is mainly performed. However, the purification method using a protein A column has an advantage in that the antibody drug can be produced with high purity at the initial stage, but has a disadvantage in that the production unit price is high because the price is 30-fold higher than that of a general ion exchange resin.

It has already been reported that protein A resin accounts for a high proportion of about 35% of the unit price of antibody drug production raw materials, and a trace amount of protein A which is eluted from the column may cause an immune or physiological reaction inside the human body. Therefore, in the case of a purification process using a protein A column, there are difficulties that the remaining amount of protein A should be monitored and removed for each process. Further, since protein A, which is a biocompatible group, has a disadvantage of being chemically weakly stable, and thus needs to be regenerated while maintaining the activity of protein A at the column regeneration stage, 1 M NaOH, which is essentially used in the cleaning process, cannot be used, so that impurities attached to the column cannot be completely removed, and accordingly, there is a limit that the number of times the column is regenerated and used is significantly reduced compared to other general chemical resins.

In order to solve such problems, many efforts have been made to remove impurities and develop a high-purity antibody using a cation exchange column, a hydrophobic column, an anion exchange column, and the like.

In particular, in a culture solution of antibodies produced using animal cells used for antibody production, particularly a CHO cell line, a large amount of not only a target antibody, but also impurities such as a host cell-derived protein (HCP) and host cell-derived DNA (HCD) are included, and further, factors for cell growth are also included. Therefore, when an antibody is purified without a protein A chromatography column, the quantity of impurities that can be removed in the early steps is of particular importance. However, in order to develop an antibody purification process capable of lowering the proportion of HCP and increasing the proportion of a main active antibody without protein A chromatography, there are difficulties in developing suitable types and sequences of chromatography, and furthermore, optimized procedures of a process.

For example, when a cation exchange column is generally used, the use of various buffers is mixed to lower the proportion of isomeric antibodies and increase the proportion of the main active antibody. Accordingly, it is difficult to collect proteins which are eluted due to a complicated process of using a plurality of buffers. In particular, since a washing process needs to use a buffer composition having a large amount of different components when using a cation exchange column and the column volume used in the washing process is very large, there is a problem in that not only the amount of buffer to be discarded in the process is considerable, but also it takes a very long time even in the preparation process.

Due to the aforementioned problems, there is a problem in that the antibody production cost and the production time are greatly increased and the yield may be low even though quality is secured.

Against this background, it is very important to provide a convenient downstream procedure for obtaining biosimilar products, particularly purified antibody fragments. In view of the problems posed by the separation and purification procedures of the related art, the present inventors sought to provide a method of purifying an antibody. The purified antibody obtained by the present invention satisfies a high yield compared to the existing process while satisfying excellent purity and activity criteria.

### DISCLOSURE

### TECHNICAL PROBLEM

The present inventors confirmed that when a sample whose pH and conductivity had been adjusted was purified under appropriate buffer conditions in a cation exchange column, a high-quality antibody preparation could be prepared by obtaining a high proportion of a main active antibody and simultaneously a high yield, thereby completing the present invention. In particular, in the present invention, the process time was reduced while reducing the large amount of buffer in the elution step used in the elution process of the cation exchange column by approximately half, thus the amount of buffer used was reduced, and the yield of produced antibody was also significantly increased, thereby completing the present invention. Accordingly, the present invention provides the invention of the following objects.

One object of the present invention provides a method of purifying an antibody, the method including: a step of loading a sample including an antibody and one or more host cell proteins (HCPs) and having a pH of 5.5 to 7.0 and a conductivity of 5 mS/cm to 8 mS/cm into an equilibrated cation exchange column, washing the cation exchange column with a washing buffer, and then eluting the antibody bound to the column with an elution buffer,

in which the washing of the cation exchange column with the washing buffer includes:
1) a step of washing the antibody with a buffer including a 10 mM to 50 mM phosphate with a pH of 5.5 to 7.0, including 0 mM to 38 mM sodium chloride, and the eluting of the antibody bound to the column with the elution buffer includes:
   1) a first elution step of eluting the antibody with a buffer including a 10 mM to 50 mM phosphate with a pH of 6.0 to 7.0, including 38 mM to 50 mM sodium chloride; and
   2) a second elution step of eluting the antibody with a buffer including a 10 mM to 50 mM phosphate with a pH of 6.0 to 7.0, including 50 mM to 60 mM sodium chloride.

Another object of the present invention is to provide an antibody prepared by the method.

### TECHNICAL SOLUTION

As one aspect to achieve the aforementioned objects, the present invention provides a method of purifying an antibody, the method including: a step of loading a sample including an antibody and one or more host cell proteins (HCPs) and having a pH of 5.5 to 7.0 and a conductivity of 5 mS/cm to 8 mS/cm into an equilibrated cation exchange column, washing the cation exchange column with a washing buffer, and then eluting the antibody bound to the column with an elution buffer.

Specifically, the present invention provides a method of purifying an antibody, the method including: a step of loading a sample including an antibody and one or more host cell proteins (HCPs) and having a pH of 5.5 to 7.0 and a conductivity of 5 mS/cm to 8 mS/cm into an equilibrated cation exchange column, washing the cation exchange column with a washing buffer, and then eluting the antibody bound to the column with an elution buffer,

in which the washing of the cation exchange column with the washing buffer includes:
1) a step of washing the antibody with a buffer including a 10 mM to 50 mM phosphate (preferably, sodium phosphate)(pH of 5.5 to 7.0) including 0 mM to 38 mM sodium chloride; and
   the eluting of the antibody bound to the column with the elution buffer includes:
   1) a first elution step of eluting the antibody with a buffer including a 10 mM to 50 mM phosphate (preferably, sodium phosphate) (pH of 6.0 to 7.0) including 38 mM to 50 mM sodium chloride; and
   2) a second elution step of eluting the antibody with a buffer including a 10 mM to 50 mM phosphate (preferably, sodium phosphate) (pH of 6.0 to 7.0) including 50 mM to 60 mM sodium chloride.

The antibody purification method according to the present invention enables purification in a higher yield even with a small amount of buffer compared to the related art, reduces the production unit price, and enables the purification process to be performed quickly, and thus has excellent advantages in the purification process of the antibody.

Specifically, the method of the present invention is a method capable of producing an antibody purification product in which a host cell-derived protein (HCP) and an isomeric antibody have been remarkably reduced without using a protein A column process in a mixture including an antibody and one or more host cell-derived proteins (HCPs). When an antibody is expressed from a host cell such as an animal cell and an antibody is obtained therefrom, a host cell protein (HCP), host cell-derived DNA (HCD), a growth factor and the like are included in addition to a target antibody in the antibody, and furthermore, an isomeric antibody (for example: an acidic isomeric antibody and a basic isomeric antibody) of the target antibody and the like may be present. Therefore, in order to make an antibody preparation, a purification process for obtaining an antibody of a desired quality by removing the aforementioned mixed impurities to increase purity is required, and in particular, a purification process capable of effectively removing a host cell protein and an isomeric antibody is required. However, in order to develop the optimization method thereof, the type of column to be used, the purification sequence, and the optimized conditions need to be investigated, so it is difficult to actually develop the process thereof.

Thus, the present invention provides a method which can remarkably reduce the levels of a host cell protein and an isomeric antibody and purify an antibody in a high yield by adjusting the pH and conductivity of a culture supernatant of host cells expressing the antibody and differentiating conditions of the washing step and the elution step in cation exchange chromatography, particularly, the properties and column volume conditions of a buffer.

As used herein, the term "sample including an antibody and one or more host cell proteins" is a culture supernatant of cells producing an antibody, a crushed product of the cells, or a sample partially purified therefrom, and refers to a sample including a target antibody to be purified and a host cell protein. The partial purification performed a filtration process, but refers to a state in which other proteins other than the antibody to be purified are also present.

Here, the sample may be adjusted so as to have a pH of 5.5 to 7.0 and a conductivity of 5.0 mS/cm to 8.0 mS/cm in order to effectively remove isomeric antibodies in the method of the present invention.

Specifically, the pH may be 5.8 to 6.2. The conductivity may be 5.0 mS/cm to 8.0 mS/cm, more specifically 6.0 mS/cm to 8.0 mS/cm, even more specifically 7.0 mS/cm to 8.0 mS/cm, and specifically about 7.0 mS/cm.

In the present invention, as a result of performing cation column chromatography under the conditions of a pH of 5.8 to 6.2 and a conductivity of 7.0 mS/cm to 8.0 mS/cm of the sample, not only was purification performed in a high yield using a small amount of buffer, but also the content of basic isomeric antibody was significantly reduced.

When a sample having such pH and conductivity is applied to cation exchange chromatography, impurities such as a host cell protein are discharged through the flow-through while the antibody to be purified is well adsorbed onto the cation exchange column, or are weakly or non-specifically bound to a cation exchange resin, and thus can be easily removed in the washing step. In addition, since the proportion of an isomeric antibody, particularly a basic isomeric antibody, can be easily reduced, a desired antibody proportion is easily adjusted.

As used herein, the term "conductivity" refers to the ability of an aqueous solution to conduct an electric current between two electrodes. An electric current flows in the solution due to ion transport. Therefore, when the amount of ions present in the aqueous solution is increased, the solution will have even higher conductivity. Since electrical conductivity is the ability of ions in a solution to carry an electric current, the conductivity of the solution may be changed by changing the ion concentration of the solution. For example, the concentration of buffer and/or salt (for example, sodium chloride, sodium acetate, or potassium chloride) in the solution may be varied to obtain a desired conductivity. Specifically, the desired conductivity may be obtained by changing the salt concentration of various buffers.

As used herein, the term "antibody" is a substance produced by stimulation of an antigen in the immune system, and refers to a substance that specifically binds to a specific antigen to cause an antigen-antibody reaction while circulating in lymphatic fluid and blood. For the purposes of the present invention, the antibody is one of the proteins for high quality purification and may be efficiently purified by the method according to the present invention.

Since the antibody generally has a higher isoelectric point than other proteins, the antibody may be primarily purified with relatively high purity when a culture supernatant is adsorbed onto a column and then eluted using an initial cation exchange resin. The isoelectric point (pI) is an average effective charge on the surface of a protein molecule, that is, a pH value at which the potential of the electric double layer of the protein molecule becomes zero, and refers to a point in which the group of the protein is dissociated, the numbers of cations and anions become equal, and thus the effective charge becomes zero. The antibody to be purified in the present invention is not limited thereto and may be an antibody having an isoelectric point of specifically 6 to 11, more specifically, 7 to 10. Further, the antibody of the present invention is not limited thereto, and specifically, may include all therapeutic antibodies typically used in the art. The antibody that may be effectively purified by the method of the present invention may be bevacizumab, which is an antibody targeting vascular endothelial growth factor A (VEGF-A). The bevacizumab antibody may have an antibody titer at a level of 1 to 3. Specifically, the bevacizumab antibody may have an antibody titer at a level of 2 to 3.

As used herein, the term "main active antibody" is a major component included in the population of antibodies of the present invention, and refers to an antibody in which some amino acids in the antibody are modified by deamination or oxidation, and thus biological activity is not lowered, that is, an antibody which is not an acidic or basic isomeric antibody. The main active antibody is the most important component for adjusting the quality of a target population of antibodies, and is an antibody with the highest biological activity among the components of the antibody.

As used herein, the term "isomeric antibody" refers to an antibody in which some amino acids of the main active antibody are modified by deamination or oxidation, and includes acidic isomeric antibodies and basic isomeric antibodies. Examples thereof include isomeric antibodies in which asparagine, among amino acids, is deaminated to become aspartate, and isomeric antibodies in which methionine, among amino acids, is oxidized to become methionine sulfate. In addition, when glutamate is present at the N-terminus of a heavy chain, the glutamate forms a pentagonal ring structure to include an isomeric antibody modified into pyruglutamate. When isomeric antibodies are produced from host cells such as CHO cells, the isomeric antibodies are included in a host cell culture solution at a high proportion, so the isomeric antibodies need to be removed by a process such as chromatography and included in the population of antibodies at a desired proportion.

Therefore, in order to prepare a high-quality population of antibodies in a host cell into which a vector including a polynucleotide encoding an antibody has been introduced, it is necessary to appropriately remove the aforementioned isomeric antibody, so that the main active antibody and the isomeric antibody are included at a desired content. Furthermore, in order to prepare a high-purity population of antibodies, impurities such as a host cell protein (HCP), host cell-derived DNA (HCD) and factors for cell growth need to be removed. Thus, in the present invention, a method of preparing an antibody in which impurities such as a host cell protein are effectively removed, in addition to adjusting the content of the isomeric antibody was developed.

As used herein, the term "host cell protein (HCP)" is a protein different from the corresponding antibody, and typically refers to a source of antibody production, that is, a protein derived from a host cell. For an antibody which may be used as a pharmaceutical, it is preferred that the HCP is excluded from an initial antibody preparation. The removed host cell protein is a concept which includes all impurities except for the antibody to be purified, and may include not only the host cell protein itself but also all of DNA derived from the host cells, factors for cell growth and the like. Therefore, when the host cell protein is removed, only the antibody to be purified may be purified with high purity.

As used herein, the term "cation exchange chromatography" refers to chromatography using a column packed with a cation exchange resin, and impurities, specifically the host cell protein and the isomeric antibody, may be removed by performing cation exchange chromatography. The cation exchange resin is a synthetic resin that serves to exchange cations in an aqueous solution with its own cations, and since an antibody has a high isoelectric point, a pH buffer solution having an isoelectric point value or less becomes positively charged. Therefore, antibody purification efficiency may be enhanced by the method of the present invention using a cation exchange resin capable of adsorbing a cation-bearing antibody, and in particular, the pH may be 5.8 to 6.2 and the conductivity may be 5.0 mS/cm to 8.0 mS/cm, specifically, 6.0 mS/cm to 8.0 mS/cm, and more specifically, 7.0 mS/cm to 8.0 mS/cm. Specifically, the conductivity may be about 7.0 mS/cm.

The present invention includes the following step as the washing step:
1) a step of washing the antibody with a buffer including a 10 mM to 50 mM phosphate (pH of 5.5 to 7.0) including 0 mM to 38 mM sodium chloride.

In the case of such a washing step, it is desirable to use the washing buffer in an amount of about 7 to 20 column volumes, specifically about 13 column volumes.

In the case of such a washing step, multiple washings may be performed using a washing buffer which increases the molar concentration of sodium chloride while maintaining the condition of a 10 mM to 50 mM phosphate at a constant molar concentration.

For example, the washing buffer includes a 10 mM to 50 mM, specifically 15 mM to 30 mM, and more specifically about 20 mM phosphate, and a primary washing may be performed using a buffer including no sodium chloride and having a pH of 5.5 to 7.0. Then, a secondary washing may be repeatedly performed using the similar buffer (for example, a buffer having different pH conditions, and the like). Next, a tertiary washing may be performed using a buffer having a pH of 6.0 to 7.0 and including 20 to 38 mM sodium chloride as a 10 mM to 50 mM, specifically 15 mM to 30 mM, and more specifically about 20 mM phosphate.

Accordingly, the step of washing the antibody with the buffer, including 1) the step of washing the antibody with a buffer including a 10 mM to 50 mM phosphate (pH of 5.5 to 7.0) including 0 mM to 38 mM sodium chloride may include the following steps:
a) a primary washing step of washing the antibody with a washing buffer including a 10 mM to 50 mM phosphate (pH of 5.5 to 7.0) not including sodium chloride;
b) a secondary washing step of washing the antibody with a washing buffer including a 10 mM to 50 mM phosphate (pH of 6.0 to 7.0) not including sodium chloride; and
c) a tertiary washing step of washing the antibody with a washing buffer including a 10 to 50 mM phosphate (pH of 6.0 to 7.0) including 20 to 38 mM sodium chloride.

Specifically, the phosphate in the steps may be a 15 mM to 30 mM, specifically about 20 mM phosphate. Sodium chloride in the steps may have a concentration of 0 mM, 0 mM and 37.5 mM, respectively.

In the case of the primary washing step, it is desirable to use the washing buffer in an amount of about 1 to 7 column volumes, specifically, about 5 column volumes.

In the case of the secondary washing step, it is desirable to use the washing buffer in an amount of about 1 to 5 column volumes, specifically about 3 column volumes.

According to specific exemplary embodiments of the present invention, the primary and secondary washing steps are washing steps so as to attach an unattached antibody to the column using a buffer having a pH of 6.0 and 6.48, respectively, and including a 20 mM phosphate.

In the case of the tertiary washing step, it is desirable to use the washing buffer in an amount of about 3 to 7 column volumes, specifically, about 5 column volumes. According to specific exemplary embodiments of the present invention, the tertiary washing step may perform washing using a 20 mM phosphate having a pH of about 6.48 and including 37.5 mM NaCl.

The present invention includes the following steps as elution steps:
1) a first elution step of eluting the antibody with a buffer including a 10 mM to 50 mM phosphate (pH of 6.0 to 7.0) including 38 mM to 50 mM sodium chloride; and
2) a second elution step of eluting the antibody with a buffer including a 10 mM to 50 mM phosphate (pH of 6.0 to 7.0) including 50 mM to 60 mM sodium chloride.

In the case of such a first elution step, it is desirable to use the buffer in an amount of about 10 to 20 column volumes, specifically about 16 column volumes.

In the case of such a second elution step, it is desirable to use the buffer in an amount of about 5 to 12 column volumes, specifically about 7 column volumes.

Specifically, the phosphate in the steps may be a 15 mM to 30 mM, specifically about 20 mM phosphate.

When a sample including the antibody to be purified and the host cell protein in the present invention is injected into a cation exchange resin, the antibody binds to the cation exchange resin, and impurities including the host cell protein pass through the column without binding (flow-through) or weakly bind to the column, so that after the sample is injected into the cation exchange resin, the weakly bound host cell protein, the isomeric antibody and the like are removed by treating the sample with a washing buffer, and then the antibody to be purified may be obtained by treating the sample with the elution buffer.

As the cation exchange resin used in the method of the present invention, those typically used in the art may be used, and although not limited thereto, specifically, carboxymethyl (CM), sulfoethyl (SE), sulfopropyl (SP), phosphate (P), sulfonate (S) or the like may be used, and more specifically, carboxylate (COO-) or sulfonate (SO₃) may be used, and among the cation exchange resins whose functional group is sulfonate, particularly, Fractogel^{™} EMD SO₃ may be used, but the cation exchange resin is not limited thereto.

The method of purifying an antibody using the Fractogel^{™} EMD SO₃ specifically may include:
(i) a step of loading a sample including a mixed solution of antibodies into a Fractogel^{™} EMD SO₃ column equilibrated with an equilibration buffer including a 10 mM to 50 mM phosphate (pH of 5.5 to 7.0);
(ii) a step of washing the column with a buffer including a 10 mM to 50 mM phosphate (pH of 5.5 to 7.0) including 0 mM to 38 mM sodium chloride;
(iii) a first elution step of eluting the antibody with a buffer including a 10 mM to 50 mM phosphate (pH of 6.0 to 7.0) including 38 mM to 50 mM sodium chloride; and
(iv) a second elution step of eluting the antibody with a buffer including a 10 mM to 50 mM phosphate (pH of 6.0 to 7.0) including 50 mM to 60 mM sodium chloride.

In particular, (ii) the step of washing the column with a buffer including a 10 mM to 50 mM phosphate (pH of 5.5 to 7.0) including 0 mM to 38 mM sodium chloride may consist of
a) a primary washing step of washing the antibody with a washing buffer including a 10 mM to 50 mM phosphate (pH of 5.5 to 7.0) not including sodium chloride;
b) a secondary washing step of washing the antibody with a washing buffer including a 10 mM to 50 mM phosphate (pH of 6.0 to 7.0) not including sodium chloride; and
c) a tertiary washing step of washing the antibody with a washing buffer including a 10 mM to 50 mM phosphate (pH of 6.0 to 7.0) including 20 to 38 mM sodium chloride.

Specifically, the phosphate in the steps may be a 15 mM to 30 mM, specifically about 20 mM phosphate.

Specifically, the method of purifying an antibody according to the present invention includes (i) a step of loading a sample including a mixed solution of antibodies into a Fractogel^{™} EMD SO₃ column equilibrated with an equilibration buffer including a 10 mM to 50 mM phosphate (pH of 5.5 to 7.0). According to specific exemplary embodiments of the present invention, equilibration may be performed using a 20 mM phosphate having a pH of about 6.0. In the case of such equilibration, it is desirable to use the buffer in an amount of about 1 to 5 column volumes, specifically about 3 column volumes.

After the step of loading the sample according to the present invention, (ii) the step of washing the column with a buffer including a 10 mM to 50 mM phosphate (pH of 5.5 to 7.0) including 0 mM to 38 mM sodium chloride may include the respective steps of the washing step described above.

Specifically, the method of purifying an antibody according to the present invention includes (iii) a first elution step of eluting the antibody with a buffer including a 10 mM to 50 mM phosphate (pH of 6.0 to 7.0) including 38 mM to 50 mM sodium chloride. According to specific exemplary embodiments of the present invention, elution may be performed using a 20 mM phosphate having a pH of about 6.48 and including 40 mM NaCl. In the case of such elution, it is desirable to use the buffer in an amount of about 10 to 20 column volumes, specifically about 16 column volumes.

Specifically, the method of purifying an antibody according to the present invention includes (iv) a second elution step of eluting the antibody with a buffer including a 10 mM to 50 mM phosphate (pH of 6.0 to 7.0) including 50 mM to 50 mM sodium chloride. According to specific exemplary embodiments of the present invention, elution may be performed using a 20 mM phosphate having a pH of about 6.48 and including 58 mM NaCl. In the case of such elution, it is desirable to use the buffer in an amount of about 5 to 12 column volumes, specifically about 7 column volumes. Unlike the related art, the present invention exhibits excellent properties in the elution step. Specifically, the elution process of the cation exchange chromatography according to the present invention has an advantage in that the buffer used in the elution step is significantly reduced. In existing known processes, it is common to perform the elution process two or more times using a buffer in an amount of, for example, at least 20 column volumes.

In contrast, in the present invention, the antibody was purified in a high yield while significantly reducing the amount (volume) of the buffer used in such an elution process and significantly reducing the host-derived protein and the isomeric antibody. Such a reduction in the amount (volume) of buffer in a bulk batch process of preparing a biosimilar has an advantage in that not only costs are significantly reduced in terms of preparation unit price, but also the time for an elution process, which generally takes a long time during the purification process, is significantly reduced. Simultaneously, the present invention has an excellent effect in that the yield of elution is maximally increased.

The method of purifying an antibody of the present invention may include a step of loading a sample including an antibody and one or more host cell proteins (HCPs) and having a pH of 5.5 to 7.0 and a conductivity of 5 mS/cm to 8 mS/cm into an equilibrated cation exchange column, washing the cation exchange column with a washing buffer, and then eluting the antibody bound to the column with an elution buffer;
a step of subjecting a filtrate recovered by the elution to ultrafiltration and diafiltration; and
a step of recovering the filtrate by allowing the filtrate to pass through a multi-layer filtration filter.

The step of subjecting the filtrate recovered by the elution to ultrafiltration and diafiltration; and the step of recovering the filtrate by allowing the filtrate to pass through the multi-layer filtration filter have the purpose of further enhancing the purity of an antibody preparation by further removing impurities such as a host cell protein, which have not been removed in the elution step using the cation exchange column. In the present step, the host cell protein may be more effectively removed using a filtration device capable of removing the host cell-derived protein, which has an isolation mechanism different from that of the cation exchange column and uses a hydrophobic reaction with electrostatic charge, and the like.

The step of subjecting the recovered filtrate to ultrafiltration and diafiltration may perform ultrafiltration and diafiltration.

Specifically, ultrafiltration may be performed. As used herein, the term "ultrafiltration" or "UF" refers to any technique for treating a solution or suspension with a semi-permeable membrane that retains macromolecules while allowing a solvent or small solute molecules to pass through. Ultrafiltration may be used to increase the concentration of macromolecules in a solution or suspension.

In addition, specifically, diafiltration may be performed. As used herein, the term "diafiltration" or "DF" is used to mean a specialized filtration category that dilutes a retentate with a solvent and refilters the retentate in order to reduce soluble permeate components. Diafiltration may induce or not induce an increase in the concentration of, for example, retained components including proteins. For example, in continuous diafiltration, the solvent is continuously added to the retentate at the same rate as the production rate of the filtrate. In this case, the volume of the retentate and the concentration of the retained components do not change during the process. Meanwhile, in discontinuous or sequentially diluted diafiltration, the ultrafiltration step involves the addition of a solvent to the retentate side; when the volume of solvent added to the retentate is equal to or greater than the volume of the resulting filtrate, the retained components will have a high concentration. Diafiltration may be used to modify pH, ionic strength, salt composition, buffer composition, or other characteristics of a solution or suspension of macromolecules.

Through such a filtration process, the content of host cell protein may be further reduced.

In the ultrafiltration and diafiltration process, it is desirable to set the condition of a pH of 5.5 to 7.0, specifically 5.8 to 6.2. An aggregate, which may be generated during the purification process, may be minimized using the pH condition described above. Further, a phosphate buffer (preferably a sodium phosphate buffer) may be used. More specifically, a 10 to 50 mM, more specifically, 20 mM phosphate buffer may be used.

The step of recovering the filtrate by allowing the filtrate to pass through a multi-layer filtration filter may be subsequently performed.

The eluted antibody eluate injected into such a multi-layer filtration filter includes both the eluted antibody eluate itself and the processed form of the eluate.

For example, the eluted antibody eluate used in the purification process of the present invention may be virus-inactivated before being allowed to pass through the multi-layer filtration filter. Such virus inactivation may be performed before or after the step of ultrafiltration and diafiltration.

Here, the virus inactivation includes rendering viruses contained in the eluate non-functional or removing viruses from the eluate. A method of rendering viruses to become non-functional or removing viruses includes heat inactivation, pH inactivation, chemical inactivation, or the like, and specifically, a pH inactivation method may be used, but the method is not limited thereto. The pH inactivation method is a method of treating viruses with a pH at which the viruses become sufficiently non-functional, and such a pH inactivation method includes a low-pH virus inactivation method, and the method may be performed by titrating the eluted antibody eluate in a pH range of 3.0 to 4.0, specifically, at a pH of 3.8, but is not limited thereto.

Further, the antibody eluate may be adjusted to a pH of 5.5 to 7.0 after the virus inactivation step and before being allowed to pass through the multi-layer filtration filter, and specifically, the pH is 6.0, but is not limited thereto.

Here, the pH may be adjusted using a buffer, and the type of buffer used in this case is not particularly limited, but specifically, a bis-Tris or phosphate buffer may be used, and more specifically, a phosphate buffer may be used.

In addition, it is desirable to adjust the pH and/or conductivity before the eluted antibody eluate is applied to the multi-layer filtration filter, and in this case, the pH of the antibody eluate is 5.5 to 7.0, and the conductivity may be adjusted to specifically, 1.2 mS/cm to 10 mS/cm, more specifically 1.3 mS/cm to 5 mS/cm.

As used herein, the term "multi-layer filtration filter" refers to a filtration device including diatomaceous earth. The multi-layer filtration filter is a filter in which a series of filters are laminated in a form in which the size of pores becomes smaller, and may have a three-dimensional matrix structure such as a maze. Examples of the action mechanism of such a multi-layer filtration filter include effective removal of a host cell protein by binding to a material such as anionic DNA and host cell proteins because the filter bears cations. As the multi-layer filtration filter, those typically used in the art may be used, but specifically, X0HC, A1HC (sold by Millipore) and the like may be used, and more specifically, X0HC may be used, but the multi-layer filtration filter is not limited thereto.

Through such a multi-layer filtration filter process, the obtained antibody filtrate may significantly reduce the content of host cell protein compared to the antibody filtrate eluted using the cation exchange column.

In the method of the present invention, after the elution step; the ultrafiltration and diafiltration step; and the step of recovering the filtrate by allowing the filtrate to pass through the multi-layer filtration filter, a step of removing the host cell protein using an anion exchange column may be further included. Specifically, a purification step of collecting a flow-through by allowing the recovered filtrate to pass through the anion exchange column may be further included.

As used herein, the term "anion exchange chromatography" refers to chromatography using a column packed with an anion exchange resin, and impurities, specifically the host cell protein, may be removed by performing anion exchange chromatography in the above step.

The anion exchange resin refers to a synthetic resin that is added to another aqueous solution and thus serves to exchange a specific anion in the aqueous solution with its own anion, and the anion exchange column may adsorb an anion-bearing protein at an isoelectric point or higher. Since an antibody has a high isoelectric point, the antibody escapes without being attached to the anion exchange resin when a neutral pH buffer is used, but impurities including a host cell protein have a low isoelectric point, and thus may be removed while being adsorbed onto the anion exchange resin, so that the purification step may be performed using the above principle.

As the anion exchange resin, those typically used in the art may be used, but the anion exchange resin is not limited thereto, and specifically, Q sepharose, quaternary aminoethyl, quaternary amine (Q), or the like may be used, and more specifically, Q Fast Flow^{™} may be used.

The host cell protein to be removed is a concept that includes all impurities except for the antibody to be purified as mentioned above, and may include not only the host cell protein itself but also all of DNA derived from the host cells, factors for cell growth and the like. Therefore, when the host cell protein is removed, only the antibody to be purified may be purified with high purity.

Further, since the anion exchange column is an efficient column for removing not only the host cell protein but also endotoxins, the anion exchange column has an advantage in that a target antibody having high purity may be purified by removing an endotoxin together with the host cell protein in the final purification step.

According to the present invention, it is possible to finally purify a high-purity and high-yield antibody from which impurities, particularly host cell proteins, have been efficiently removed, when the antibody purification method including the steps mentioned above is performed.

In particular, the method according to the present invention has an excellent advantage in the antibody preparation process in that the use of a large amount of buffer used in the related art is significantly reduced to achieve a significant reduction in terms of preparation unit price and preparation time.

After the final purification, the content of the host cell protein may be specifically 0.001 to 10 ppm and more specifically 0.01 to 5 ppm.

Therefore, as another aspect of the present invention, the present invention provides an antibody prepared by the method.

### ADVANTAGEOUS EFFECTS

When the method of purifying an antibody according to the present invention is used, it is possible to prepare a target antibody with high purity and high quality by removing impurities without using an expensive protein A column. In particular, the present invention has an excellent effect in that the amount (volume) of the buffer used in the elution process is significantly reduced, and simultaneously, the antibody is purified in a high yield.

### DESCRIPTION OF DRAWINGS

FIG. 1 illustrates an elution profile of the cation exchange chromatography step according to an exemplary embodiment of the present invention.
FIG. 2 is a CE-HPLC analysis graph of an eluate obtained according to an exemplary embodiment of the present invention.
FIG. 3 is a graph showing the suitable pH conditions of the ultrafiltration and diafiltration according to an exemplary embodiment of the present invention.

### MODES OF THE INVENTION

Hereinafter, the present invention will be described in detail through the Examples. However, the following Examples are only for exemplifying the present invention, and the present invention is not limited by the following Examples.

### Example 1: Bevacizumab pre-treatment step

A bevacizumab antibody was expressed by culturing recombinant CHO cells expressing the bevacizumab antibody. It was confirmed that the bevacizumab antibody according to exemplary embodiments of the present invention had an antibody titer at a level of 2 to 3, and the antibody was used in the experiment of the present invention.

In order to adsorb the antibody onto a cation exchange column, an experiment was performed by adding a 1 M glycine HCI (pH of 3.0) buffer to a culture solution to adjust the pH to 6.0 and adjusting the conductivity to 6.5 mS/cm to 8.5 mS/cm.

The amount of 1 M glycine-HCI (pH of 3.0) used for the pre-treatment of the culture solution for each test group, the amount of tertiary purified water added, and the loaded sample conditions after the pre-treatment are as follows in the following Table 1.

**[Table 1]**

| Conductivity | Culture solution | 1 M glycine-HCI (pH3.0) | Tertiary purified water | Loaded sample after pre-treatment | |
|---|---|---|---|---|---|
| | | | | pH | Conductivity |
| 6.5 mS/cm | 400 mL | 17.40 mL | 498.15 mL | 6.02 | 6.48 |
| 7.0 mS/cm | 400 mL | 17.40 mL | 450.15 mL | 6.02 | 7.01 |
| 7.5 mS/cm | 400 mL | 17.56 mL | 354.53 mL | 6.00 | 7.5 |
| 8.0 mS/cm | 454.97 mL | 20.51 mL | 363.57 mL | 6.01 | 8.02 |
| 8.5 mS/cm | 400 mL | 17.42 mL | 272.99 mL | 6.03 | 8.45 |

### Example 2: Cation exchange column chromatography

In the present example, a Fractogel^{™} EMD SO₃ column was used as a cation exchange column, and the process was as follows.

For equilibration, a 20 mM phosphate buffer (pH of 6.0) was flowed in an amount of 3 column volumes to equilibrate the column. Then, the supernatant for which the pre-treatment of Example 1 had been completed, was loaded at or below the adsorption capacity of SO₃. The loading amount was 30 g or less of protein per 1 L resin volume, and the loading rate was 150 cm/hr.

Then, a three-step washing step and a two-step elution step were performed under the conditions shown in the following Table 2.

**[Table 2]**

| | Buffer | Column volumes |
|---|---|---|
| Washing 1 step | 20 mM phosphate buffer, pH 6.0, | 5 |
| Washing 2 step | 20 mM phosphate buffer, pH 6.48, | 3 |
| Washing 3 step | 20 mM phosphate buffer including 37.5 mM NaCl, pH 6.48, 5.8 mS/cm | 5 |
| Elution 1 step | 20 mM phosphate buffer including 40 mM NaCl, pH 6.48, 6.2 mS/cm | 16 |
| Elution 2 step | 20 mM phosphate buffer including 58 mM NaCl, pH 6.48, 7.9 mS/cm | 7 |

As can be confirmed above, in the present invention, the column volumes of the elution 1 step and the elution 2 step corresponding to the washing 4 step and the elution 1 step, respectively, of the following Comparative Example were reduced to 16 column volumes and 7 column volumes, respectively.

Meanwhile, for comparison, a Comparative Example was set under the conditions of the following Table 3 in which the column volumes in the washing 4 step (corresponding to the elution 1 step in the Examples) and the elution 1 step (corresponding to the elution 2 step in the Examples) were set to 30 column volumes and 20 column volumes, respectively.

**[Table 3]**

| | Buffer | Column volumes |
|---|---|---|
| Washing 1 step | 20 mM phosphate buffer, pH 6.0, | 5 |
| Washing 2 step | 20 mM phosphate buffer, pH 6.48, | 3 |
| Washing 3 step | 20 mM phosphate buffer including 37.5 mM NaCl, pH 6.48, 5.8 mS/cm | 5 |
| Washing 4 step | 20 mM phosphate buffer including 40 mM NaCl, pH 6.48, 6.2 mS/cm | 30 |
| Elution 1 step | 20 mM phosphate buffer including 58 mM NaCl, pH 6.48, 7.9 mS/cm | 20 |

In particular, in the case of the above Comparative Example, the column volume is used in the same form as the method actually used in the invention of Korean Patent No. 10-1569783.

### Example 3: Confirmation of yield and charge variant content by difference in pre-treatment conductivity

The changes in step yield and charge variant content were confirmed by subjecting the pre-treated bevacizumab sample prepared according to the conductivity according to Example 1 to cation exchange chromatography under the conditions of Example 2.

The results are shown in Tables 4 and 5.

**[Table 4]**

| Conductivity | Loaded protein amount | Eluate volume | Total protein recovery amount | Step yield |
|---|---|---|---|---|
| 6.5 mS/cm | 409.5 mg | 167.26 mL | 348.0 mg | 84.9% |
| 7.0 mS/cm | 402.7 mg | 166.56 mL | 389.1 mg | 96.7% |
| 7.5 mS/cm | 400.7 mg | 166.56 mL | 373.1 mg | 93.2% |
| 8.0 mS/cm | 422.1 mg | 164.72 mL | 388.6 mg | 92.0% |
| 8.5 mS/cm | 394.8 mg | 123.10 mL | 117.8 mg | 29.8% |

**[Table 5]**

| Experimental group | Acidic peak (%) | Main peak (%) | Basic peak (%) | Total basic (%) |
|---|---|---|---|---|
| 6.5 mS/cm | 23.1 | 72.9 | 1.6 | 4.0 |
| 7.0 mS/cm | 20.6 | 75.8 | 0.8 | 2.1 |
| 7.5 mS/cm | 21.5 | 74.8 | 0.9 | 2.5 |
| 8.0 mS/cm | 16.7 | 79.4 | 1.2 | 3.0 |
| 8.5 mS/cm | 12.4 | 79.4 | 3.6 | 8.1 |

Table 4 shows the results of confirming the change in yield according to the difference in pre-treatment conductivity. As can be confirmed in Table 4, it could be confirmed that a step yield of 90.0% or more at 7.0 mS/cm to 8.0 mS/cm was exhibited, and it was confirmed that the best step yield was exhibited particularly at 7.0 mS/cm.

Further, Table 5 shows the results of comparing the charge variant content according to the difference in pre-treatment conductivity. As can be confirmed in Table 5, a main peak similar to a control drug Avastin, and acidic isomer and basic isomer peaks at 7.0 mS/cm to 8.0 mS/cm according to the present invention, and particularly, a main peak, which is most similar to a control drug Avastin, and acidic isomer and basic isomer peaks at 7.0 mS/cm were exhibited.

In particular, when the elution process was performed through the use of a small amount of buffer with the change in conductivity as described above, excellent step yield and low basic isomer content could be confirmed.

### Example 4: Confirmation of increase in yield according to decrease in column volume

Under the conditions of Example 2, cation exchange chromatography was performed on the following sample having a conductivity of 7.0 mS/cm.

In the case of the pre-treated sample of Example 1, the sample concentration was 1.04 mg/ml, the loading volume was 162 ml, and the total protein dose was 168.8 mg.

In the purification process, the conditions of CV: 5 mL (pre-packed), bed height: 10.0 cm, flow rate: 0.67 mL/min or 0.84 mL/min were used.

Meanwhile, changes in yield were confirmed by testing the conditions of the Comparative Example in Table 3 together in the same manner as the above conditions.

The results are shown in Table 6.

**[Table 6]**

| Process | Yield (%) | Acidic peak (%) | Main peak (%) | Basic peak (%) | Total basic (%) |
|---|---|---|---|---|---|
| Example 2 (Repetition 1) | 66.5 | 8.9 | 85.3 | 4.1 | 5.9 |
| Example 2 (Repetition 2) | 66.5 | 9.1 | 85.1 | 4.1 | 5.9 |
| Example 2 (Repetition 3) | 66.9 | 8.8 | 85.3 | 4.2 | 5.9 |
| Comparative Example | 48.1 | 6.8 | 87.2 | 3.9 | 6.0 |

As confirmed above, the yield during the application of the purification process of the Comparative Example was only about 50%. Meanwhile, when the method according to the present invention was used, a high yield of about 60% or more was shown, although the time of the washing process and the elution process and the amount of buffer used were significantly reduced. In addition, it was confirmed that a charge variant content was exhibited at a level suitable for biosimilar preparation even in terms of charge variant content.

### Example 5: Virus inactivation

Viruses were inactivated at a pH of 3.8 for 1 hour by adding a 1 M glycine-HCI (pH of 3.0) buffer to the eluate subjected to the cation exchange chromatography according to Example 2. After the inactivation was completed, the sample was allowed to pass through a 0.2 µm filter, and then the pH of the sample was adjusted to 6.0 by adding a 1 M Tris-HCI (pH of 9.0) buffer.

### Example 6: Ultrafiltration and diafiltration process

In consideration of the sample volume, a micro-centricon (0.5 mL) was used in the ultrafiltration and diafiltration (UF/DF1) process, and the process was performed in triplicate under each condition.

Amicon Ultra 0.5 mL Ultracel was used as the micro-centricon, and an Eppendorf centrifuge was used as a centrifuge used for concentration and buffer exchange. In order to adjust an initial concentrated concentration of 20 mg/mL, 400 µl (5.13 mg/mL) of UF/DF1 injection sample was put into the micro-centricon, and centrifugation was performed at 5,000 rpm for 10 minutes to concentrate the sample to 100 µl. The sample was diluted 2-fold by adding 100 µl of the buffer at each pH in Table 7 to the sample concentrated to 100 µl, and then centrifuged again at 5,000 rpm for 5 minutes to concentrate the sample to 100 µl. UF/DF1 was completed by performing the above process 7 times.

For the most accurate experiment, micro-centricon, UF/DF1 injection sample, and post-UF volumes were all measured by weight.

**[Table 7]**

| Name | Buffer composition | Measured values | |
|---|---|---|---|
| | | pH | Cond. (mS/cm) |
| Experiment 1 | 20 mM Na-phosphate pH 6.0 | 5.988 | 1.61 |
| Experiment 2 | 20 mM Na-phosphate pH 6.5 | 6.519 | 1.89 |
| Experiment 3 | 20 mM Na-phosphate pH 7.0 | 7.000 | 2.53 |
| Experiment 4 | 50 mM Tris-HCI, 20 mM NaCl pH 7.0 | 6.997 | 6.36 |
| Experiment 5 | 50 mM Tris-HCI, 20 mM NaCl pH 7.5 | 7.504 | 5.84 |

For the application of an integration event for the SE-HPLC analysis, the aggregate content was analyzed by applying the integration event defined in the SOP.

The results are shown in Table 8.

**[Table 8]**

| Name | No. | SE-HPLC (%) | |
|---|---|---|---|
| | | Aggregate | AVG. Aggregate |
| 50 mM Tris-HCI, 20 mM NaCl pH 7.5 | 1 | 5.24 | 5.44 |
| | 2 | 4.76 | |
| | 3 | 6.33 | |
| 50 mM Tris-HCI, 20 mM NaCl pH 7.0 | 1 | 4.82 | 4.68 |
| | 2 | 4.64 | |
| | 3 | 4.59 | |
| 20 mM Na-phosphate pH 7.0 | 1 | 3.95 | 3.70 |
| | 2 | 3.57 | |
| | 3 | 3.54 | |
| 20 mM Na-phosphate pH 6.5 | 1 | 3.09 | 2.98 |
| | 2 | 2.92 | |
| | 3 | 2.95 | |
| 20 mM Na-phosphate pH 6.0 | 1 | 1.82 | 1.83 |
| | 2 | 1.83 | |
| | 3 | 1.84 | |

As a result of the aggregation content analysis under each pH condition, it was confirmed that the aggregation content increased with the change in pH as can be confirmed in Table 8.

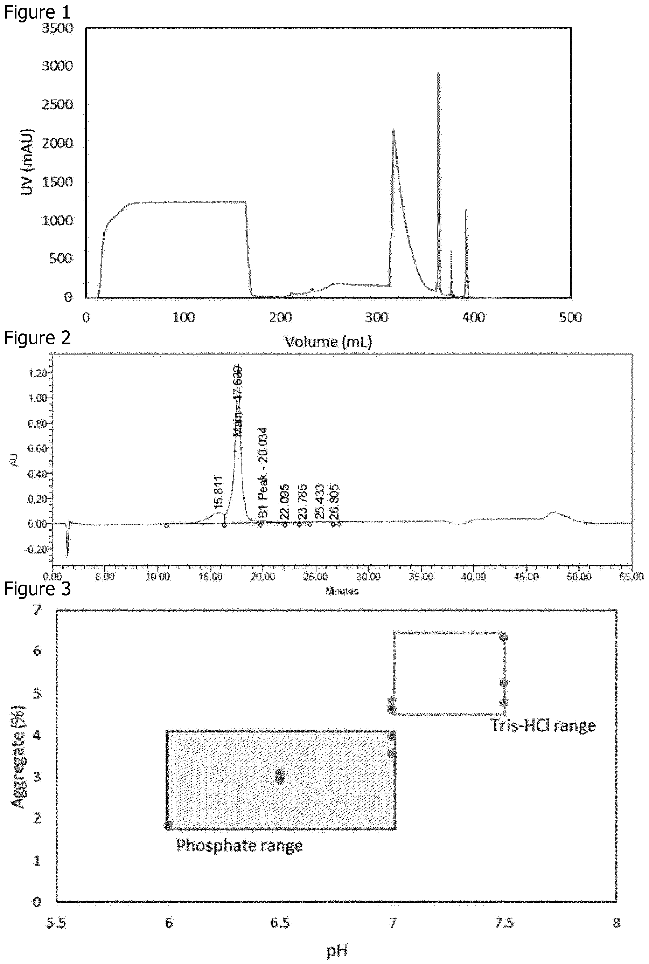

In addition, as can be confirmed in FIG. 3, as a result of confirming the linearity with respect to an independent variable (pH) in order to confirm the significance of the increase in aggregate content in the UF/DF1 process according to the increase in pH, a linear equation of Y = 2.4x-12.6 was confirmed, and R2 was confirmed to be 0.88. Since the slope value (2.4) has a positive value, it was confirmed that the dependent variable (aggregate content) on the Y-axis increases as the independent variable (pH) on the X-axis increases.

Through the results, it was determined that it is difficult for the conditions of pH 7.5 or higher using a Tris-HCI buffer to be applied to the process because the aggregate content increases in the UF/DF1 step. In contrast, it was confirmed that it was suitable to apply the condition of 20 mM Na-phosphate at pH 6.0.

### Example 7: Multi-layer filtration filtering process

The multi-layer filtration filter may reduce host-derived DNA (HCD), host-derived protein (HCP), and the like due to its electrostatic properties and the like. Thus, an XOHC-type multi-layer filtration filter was prepared, and tertiary purified water and a 20 mM sodium phosphate (pH of 6.0) buffer were flowed for substitution and equilibration. Then, after viruses were inactivated, the sample of Example 6 that had been subjected to a filtration process was substituted with a 20 mM sodium phosphate (pH of 6.0) buffer, and then filtered at a flow rate of 100 LMH(liter/m2/hour) by flowing the substituted sample into the multi-layer filtration filter. All the filtered samples were recovered and used as samples for the following anion exchange column chromatography purification.

### Example 8: Anion exchange column chromatography

Since the anion exchange column adsorbs anion-bearing proteins at the isoelectric point or more, in the case of an antibody with an isoelectric point of 7 or less (in the case of bevacizumab, its isoelectric point is 8.3), when a buffer with a neutral pH is used, this antibody does not adhere to the anion exchange resin and escapes into the flow-through. Thus, the following experiments were conducted in order to investigate the anion exchange resin and buffer solution conditions suitable for the preparation process of the present invention.

Specifically, in the present example, purification was performed using quaternary amine series Q sepharose^{™} Fastflow, which is frequently used as an anion exchange resin on a production scale.

First, a sample for loading into the anion exchange resin was prepared to have a pH of 6.0 and a conductivity of 1.4 mS/cm by completing cation exchange column chromatography for a culture supernatant and virus inactivation as in the Examples, subjecting the sample to primary ultrafiltration, and then substituting the sample with a 50 mM sodium phosphate (pH of 6.0) equilibration buffer.

The loading amount was 30 g or less of protein per 1 L resin volume, the loading and elution rate was 150 cm/hr, and a column flow-through was collected when the absorbance at A280 nm increased. The column was regenerated with 1 M NaCl and then equilibrated with an equilibration buffer.

The step yield under each pH condition was confirmed by subjecting the bevacizumab sample prepared according to Example 7 to anion exchange chromatography by the method as described above.

The results are shown in Table 9.

**[Table 9]**

| pH Condition | Step yield (%) |
|---|---|
| 6.0 | 96.6% |
| 7.0 | 95.4% |
| 7.5 | 93.2% |
| 8.0 | 90.3% |
| 8.5 | 62.8% |

Table 9 shows the results of confirming the change in step yield according to the difference in pH condition. As can be confirmed in Table 9, it could be confirmed that when anion exchange chromatography was performed, a step yield of 90.0% or more in a pH range of 6.0 to 8.0 was exhibited, and particularly, it was confirmed that the best step yield was exhibited at a pH of 6.0.

From the foregoing description, it will be understood by those skilled in the art to which the present invention pertains that the present invention can be implemented in other concrete forms without modifying the technical spirit or essential features of the present invention. In this regard, it should be understood that the above-described embodiments are only exemplary in all aspects and are not restrictive. The scope of the present invention is represented by the claims to be described below rather than the detailed description, and it should be interpreted that the meaning and scope of the claims and all the changes or modified forms derived from the equivalent concepts thereto fall within the scope of the present invention.

## Claims

1. A method of purifying an antibody, the method comprising: a step of loading a sample comprising an antibody and one or more host cell proteins (HCPs) and having a pH of 5.5 to 7.0 and a conductivity of 5 mS/cm to 8 mS/cm into an equilibrated cation exchange column, washing the cation exchange column with a washing buffer, and then eluting the antibody bound to the column with an elution buffer,
wherein the washing of the cation exchange column with the washing buffer includes:
1) a step of washing the antibody with a buffer comprising a 10 mM to 50 mM phosphate with a pH of 5.5 to 7.0, comprising 0 mM to 38 mM sodium chloride, and
the eluting of the antibody bound to the column with the elution buffer includes:
1) a first elution step of eluting the antibody with a buffer comprising a 10 mM to 50 mM phosphate with a pH of 6.0 to 7.0, comprising 38 mM to 50 mM sodium chloride; and
2) a second elution step of eluting the antibody with a buffer comprising a 10 mM to 50 mM phosphate with a pH of 6.0 to 7.0, comprising 50 mM to 60 mM sodium chloride.

2. The method of claim 1, wherein 1) the step of washing the antibody with a buffer comprising a 10 mM to 50 mM phosphate with a pH of 5.5 to 7.0, comprising 0 mM to 38 mM sodium chloride comprises the following three steps:
a) a primary washing step of washing the antibody with a washing buffer comprising a 10 mM to 50 mM phosphate having a pH of 5.5 to 7.0 that does not comprise sodium chloride;
b) a secondary washing step of washing the antibody with a washing buffer including a 10 mM to 50 mM phosphate having a pH of 5.5 to 7.0 that does not comprise sodium chloride; and
c) a tertiary washing step of washing the antibody with a washing buffer comprising a 10 to 7.0 mM phosphate having a pH of 6.0 to 7.0 that comprises 20 to 38 mM sodium chloride.

3. The method of claim 1, wherein in the first elution step, the antibody is treated with 10 to 20 column volumes of the buffer.

4. The method of claim 1, wherein in the second elution step, the antibody is treated with 5 to 12 column volumes of the buffer.

5. The method of claim 1, wherein the antibody has an isoelectric point of 6 to 11.

6. The method of claim 1, wherein the antibody is bevacizumab.

7. The method of claim 1, wherein a functional group of the cation exchange column is selected from the group consisting of carboxymethyl (CM), sulfoethyl (SE), sulfopropyl (SP), phosphate (P) and sulfonate (S).

8. The method of claim 7, wherein a functional group of the cation exchange column is Fractogel^{™} EMD SO₃.

9. The method of claim 1, further comprising:
a step of subjecting a filtrate recovered by the elution step to ultrafiltration and diafiltration; and a step of recovering the filtrate by allowing the filtrate to pass through a multi-layer filtration filter.

10. The method of claim 9, further comprising: removing the host cell protein using an anion exchange column after the step of recovering the filtrate by allowing the filtrate to pass through a multi-layer filtration filter.
